# EUROPÄISCHE PATENTSCHRIFT

(11) **EP 0 509 309 B1**
(45) Veröffentlichungstag und Bekanntmachung des Hinweises auf die Patenterteilung: **30.11.1994**
(21) Anmeldenummer: 92105527.3
(22) Anmeldetag: 31.03.1992
(51) Int. Cl.: C07C 209/78, C07C 211/50

(54) **Verfahren zur Herstellung von mehrkernigen aromatischen Polyaminen**
Process for the preparation of polynuclear aromatic polyamines
Procédé pour la préparation de polyamines aromatiques polynucléaires

(30) Priorität: 13.04.1991 DE 4112131
(43) Veröffentlichungstag der Anmeldung: 21.10.1992
(73) Patentinhaber: BAYER AG, 51368 Leverkusen (DE)
(72) Erfinder: Knöfel, Hartmut, Dr., W-5068 Odenthal (DE); Brockelt, Michael, W-5090 Leverkusen 3 (DE); Petinaux, Marcel, Dr., Pittsburgh, PA 15241 (US); Uchdorf, Rudolf, Dipl.-Ing., W-4150 Krefeld 11 (DE); Schal, Hans-Peter, Dr., W-4047 Dormagen 1 (DE)

(56) Entgegenhaltungen:
- EP-A- 0 288 892
- EP-A- 0 337 205

## Beschreibung

Die Erfindung betrifft eine verbesserte Ausführungsform des Verfahrens der Herstellung von mehrkernigen aromatischen Polyaminen durch Kondensation von Anilin mit Formaldehyd in Gegenwart von Wasser und sauren Katalysatoren und Aufarbeitung des Reaktionsgemisches durch Extraktion mit einem hydrophoben Lösungsmittel unter Wiederverwendung des in der wäßrigen Phase der Extraktion anfallenden Säurekatalysators.

Es ist bereits bekannt, bei der Herstellung von mehrkernigen aromatischen Polyaminen durch Kondensation von Anilin mit Formaldehyd in Gegenwart von Wasser und sauren Katalysatoren das anfallende wäßrige Reaktionsgemisch durch Extraktion mit einem hydrophoben Lösungsmittel aufzuarbeiten und den bei der Extraktion in der wäßrigen Phase anfallenden Säurekatalysators wiederzuverwenden (vgl. z.B. DE-OS 2 238 920, DE-OS 2 343 658, DE-OS 2 356 828, DE-OS 2 500 573, DE-OS 2 500 574 oder DE-OS 2 528 694).

Der wesentliche Vorteil dar Verfahren dieser Vorveröffentlichungen ist darin zu sehen, daß auf eine Neutralisation des Katalysators verzichtet werden kann, da er bei der extraktiven Aufarbeitung des sauren Reaktionsgemischs in der wäßrigen Phase anfällt, die als solche an den Prozeßanfang zurückgeführt und wiederverwendet wird. Außerdem gestatten bestimmte Varianten dieses bekannten Prinzips, wie sie beispielsweise in DE-OS 2 500 574 oder 2 528 694 beschrieben sind, die gezielte Herstellung von Polyamingemischen mit wahlweise erhöhtem oder erniedrigtem Gehalt an 2,4'-Isomeren. Im übrigen entsprechen die Verfahrensprodukte der Vorveröffentlichungen bezüglich ihrer Eignung als Vorprodukt zur Herstellung von Polyisocyanaten den klassischen Polyaminen der Diphenylmethanreihe, die unter Neutralisation des eingesetzten Säurekatalystors hergestellt worden sind. Dies bedeutet, daß das Eigenschaftsniveau der aus derartigen Polyisocyanatgemischen der Diphenylmethanreihe hergestellten Polyurethanschaumstoffe in beiden Fällen annähernd das gleiche ist. Es ist aber als ein Nachteil der Verfahren der genannten Vorveröffentlichungen anzusehen, daß zur Aufarbeitung der Verfahrensprodukte durch Extraktion ausschließlich für diesen Zweck beträchtliche Mengen an hydrophobem Lösungsmittel und Anilin eingesetzt werden müssen, was selbstverständlich auf einen beträchtlichen Destillationsaufwand und damit Energieverbrauch bei der destillativen Aufarbeitung der organischen Phase hinausläuft.

Einen gewissen Fortschritt gegenüber dem obengenannten Stand der Technik unter Erhaltung von dessen Vorteilen stellen später veröffentlichte Verfahren des Standes der Technik dar, wie sie in EP O 288 892 und EP O 337 205 beschrieben werden.

Die Vorteile dieser Verfahren betreffen insbesondere die Produktqualität und Produktenflexibilität neben ökonomischen Verbesserungen. Sie beruhen im wesentlichen auf der Mitverwendung eines hydrophoben Lösungsmittels auch während des Reaktionsablaufs. Dabei resultieren weitere Vorteile aus der teilweisen Doppelfunktion diese hydrophoben Lösungsmittels als Bestandteil sowohl des Reaktionsgemisches als auch das Extraktionsmittels.

Insgesamt zeichnen sich der Stand der Technik gemäß EP O 288 892 und EP O 337 205 folgende Vorteile aus:
- Der eingesetzte Säurekatalysator wird wiederverwendet und nicht durch Neutralisation vernichtet.
- Die bei der destillativen Aufarbeitung der, die Verfahrensprodukte enthaltenden, organischen Phase als Destillat anfallenden Gemische, können ohne weitere destillative Auftrennung in ihre Bestandteile als solche, gegebenenfalls nach Zugabe von weiterem Anilin, als Extraktionsmittel für die wäßrige Phase in der Produktextraktionsstufe wiederverwendet werden.
- Die Verfahren sind bezüglich der Homologenverteilung in den Verfahrensprodukten (Verhältnis von Diaminen zu höherfunktionellen Polyaminen) innerhalb weiter Grenzen variierbar.
- Die Verfahren gestatten die Herstellung von Polyaminen der Diphenylmethanreihe mit einem relativ erhöhten Gehalt an 2,4'-Diaminodiphenylmethan bei gleichzeitig geringem Gehalt an stets unerwünschtem 2,2'-Diaminodiphenylmethan.

Als Nachteil dieser Verfahren erwiesen sich die im Zusammenhang mit der vorteilhaften hohen Selektivität der zweiphasigen Reaktionsführung einhergehenden verlängerten Reaktionszeiten, insbesondere in der zweiten Umlagerungsstufe, bis zur vollständigen Umlagerung in die gewünschten Endprodukte. Daraus resultiert die Gefahr der unvollständigen Umlagerung mit der sich ergebenden Problematik für die Qualität der Endprodukte und insbesondere der abgeleiteten Polyisocyanate.

Unvollständige Umlagerung bedeutet aber das Vorhandensein von Kondensationszwischenprodukten z.B. vom N-Aminobenzyltyp im Reaktionsgemisch am Ende der eigentlichen Reaktion. Bei der Aufarbeitung des Reaktionsgemisches zur Isolierung der Verfahrensendprodukte gemäß den Verfahren des Standes der Technik gelangen die Zwischen- und Nebenprodukte in die Verfahrensendprodukte, was mit deutlichen Qualitätseinbußen, insbesondere bei den resultierenden Polyisocyanaten verbunden ist. Um letzteres durch Sicherstellung von vollständiger Umlagerung am Ende der Reaktion zu vermeiden, muß bei den zitierten Verfahren des Standes der Technik, insbesondere bei kontinuierlicher Betriebsweise, mit einem erheblichen Aufwand bei der Reaktionszeit bzw. dem Reaktionsvolumen und/oder der Reaktionstemperatur, insbesondere in der letzten Umlagerungsstufe, gegengesteuert werden.

Bei EP O 288 892 wird die organische Phase am Ende der Umlagerungsreaktion abgetrennt und zur Gewinnung der Verfahrensendprodukte mitsamt evtl. vorhandener Kondensationszwischenprodukte eingesetzt.

Letztere sind in dieser organischen Phase (E) gegenüber der wässrigen Phase (F) angereichert und gelangen mit dieser über die Aufarbeitungsstufe(n) in das Verfahrensprodukt.

Bei EP O 337 205 werden aus der organischen Phase nach ihrer Abtrennung am Ende der Umlagerungsreaktion die darin enthaltenen Kondensationsprodukte insgesamt durch Extraktion in eine wäßrige Phase überführt und in den Reaktionsablauf rezykliert. Die verbleibende organische Phase wird anschließend an anderer Stelle als Extraktionsmittel für die Verfahrensprodukte eingesetzt. Bei der Extraktion mit der wässrigen Phase konzentrieren sich evtl. vorhandene Kondensationszwischenprodukte zunächst in dieser organischen Phase infolge der Selektivität dieses Vorgangs auf, um schließlich als letzte Komponenten des Kondensationsproduktgemisches in die wäßrige Phase überzugehen. Die Extraktion muß daher mit erheblichem Aufwand möglichst quantitativ durchgeführt werden, damit die verbleibende, extrahierte organische Phase als Extraktionsmittel für Verfahrensendprodukte in der Hauptextraktionsstufe ohne Nachteil nochmals verwendet werden kann.

Es war die der Erfindung zugrunde liegende Aufgabe, ein neues verbessertes Verfahren zur Herstellung von mehrkernigen aromatischen Polyaminen aus Anilin und Formaldehyd zur Verfügung zu stellen, welches die Vorteile des bekannten Standes der Technik in sich vereinigt und unter Vermeidung der Nachteile die Herstellung von qualitativ weiter verbesserten Produkten bei gleichzeitig reduziertem Verfahrensaufwand und verbesserter Sicherheit bei der erzielten Produktqualität ermöglicht.

Durch selektive Abtrennung und interne Rückführung von Kondensationszwischenprodukten in den Reaktionsablauf vor der Gewinnung und Isolierung der eigentlichen Verfahrensprodukte ergaben sich folgende Verbesserungen:
1. Die isolierten Verfahrensprodukte weisen einen äußerst geringen Gehalt an Kondensationszwischenprodukten auf, der in allen Fällen deutlich geringer ist als in den Reaktionsendprodukten am Ende der eigentlichen Reaktion, daher kann
2. in den Reaktionsendprodukten ein höherer Gehalt an Kondensationszwischenprodukten toleriert werden als bei den Verfahren des Standes der Technik ohne Beeinträchtigung der Verfahrensendprodukte bzw. der Folgeprodukte.
3. Darüber hinaus bietet das erfindungsgemäße Verfahren zusätzliche Sicherheit für die Qualität der Endprodukte gegen "Durchschlagen" von Kondensationszwischenprodukten in die isolierten Endprodukte, falls durch Verfahrensschwankungen und -störungen am Ende der Umlagerungsreaktion erhöhte Werte dieser Zwischenprodukte auftreten.
4. Die isolierten Verfahrensprodukte nach der Aufarbeitung weisen stets einen geringeren Gehalt an 2,2'-Diaminodiphenylmethan auf als die Reaktionsendprodukte.
5. Die isolierten Verfahrensendprodukte weisen ggf. auch einen geringeren Gehalt an 2,4'-Diaminodiphenylmethan auf als die Reaktionsendprodukte.
6. Der Gehalt der Verfahrensendprodukte an 2,4'-Diaminodiphenylmethan kann in weiten Grenzen gesteuert werden unabhängig von den eigentlichen Reaktionsparametern wie Anilin-Formaldehyd-(= Kondensationsverhältnis, Amin-Katalysatorverhältnis (= Protonierungsgrad), Phasenverhältnis von organischer zu wäßriger Phase während der Reaktion.
7. Die isolierten Verfahrensprodukte weisen ggf. auch einen geringeren Gehalt an ortho-substituierten höheren Homologen des Diaminodiphenylmethans auf als die Reaktionsprodukte.

Gegenstand der Erfindung ist ein Verfahren zur Herstellung von mehrkernigen aromatischen Polyaminen durch Umsetzung von Anilin mit Formaldehyd in Gegenwart von Wasser und sauren Katalysatoren in einer ein- oder zweistufigen Reaktion innerhalb des Temperaturbereichs von 0 bis 180°C, gegebenenfalls unter Vorschaltung einer Aminal-Vorstufe, in welcher die Bildung von N,N'-disubstituiertem Aminal stattfindet, welches dann ein- oder mehrstufig in Gegenwart von Säurekatalysator innerhalb des Temperaturbereichs von 0 bis 180°C in das gewünschte Endprodukt überführt wird, Aufarbeitung des resultierenden Reaktionsgemischs durch Extraktion mit einem Anilinhaltigen hydrophoben Lösungsmittel in einer Produktextraktionsstufe (8) destillative Auftrennung der hierbei anfallenden organischen Phase in (i) ein aus Anilinhaltigem Lösungsmittel bestehendes Destillat, welches gegebenenfalls nach Zugabe von Frischanilin erneut bei der Extraktion eingesetzt wird und (ii) einen im wesentlichen aus Verfahrensprodukt bestehenden Destillationsrückstand und Rückführung von bei der Extraktion anfallender, den Säurekatalysator enthaltender wäßriger Phase unter Wiederverwendung des in ihr enthaltenen Katalysators, unter Entfernung des bei der Kondensationsreaktion entstehenden Kondensationswassers und des in das System mit der wäßrigen Lösung des Formaldehyds eingebrachten Wassers in einem der Aminalvorstufe nachgeschalteten und der ersten Reaktionsstufe vorgeschalteten Wasserabscheider und/oder in einem der Extraktionsstufe nachgeschalteten Verdampfer, dadurch gekennzeichnet, daß man
a) den in Form einer wäßrigen Lösung zum Einsatz gelangenden Formaldehyd in einer Aminal-Vorstufe (3) mit einer aus Anilin, gegebenenfalls Anilin-Formaldehyd-Kondensationsprodukten und aus hydrophobem Lösungsmittel bestehenden organischen Phase und/oder in der ersten Reaktionsstufe (5) mit einer aus Anilin, gegebenenfalls Anilin-Formaldehyd-Kondensationsprodukten und aus hydrophobem Lösungsmittel bestehenden organischen Phase und recyclisierter, den Katalysator in Form von Aminsalzen enthaltender wäßriger Phase durch Vermischen zur Reaktion bringt,
b) das so erhaltene zweiphasige Reaktionsgemisch nach Beendigung der Reaktion in einem der Produktextraktionsstufe (8) vorgeschalteten Phasenscheider (7) in eine wäßrige Phase und eine organische Phase auftrennt,
c) die im Phasenscheider (7)anfallende organische Phase in einer der Produktextraktionsstufe (8) nachgeschalteten Nachextraktionsstufe (10) mit einem Teil der in der Produktextraktionsstufe (8) anfallenden, weitgehend vom Reaktionsprodukt befreiten wäßrigen Phase extrahiert,
d) die in der Nachextraktionsstufe (10) anfallende mit dem der im Phasenscheider (7) anfallenden organischen Phase angereicherte wäßrige Phase vollständig oder zumindest teilweise in den Reaktionsprozess nach beendeter Umlagerung (6) und vor der Hauptextraktionsstufe (8) zurückführt und den gegebenenfalls verbleibenden Rest der wäßrigen Phase vor beendeter Umlagerung (6) zurückführt,
e) die in der Nachextraktionsstufe (10) anfallende, aus Anilin, gegebenenfalls Anilin-FormaldehydKondensationsprodukten und aus hydrophobem Lösungsmittel bestehende organische Phase an den Prozessanfang zurückführt und gemäß a) umsetzt,
f) die im Phasenscheider (7) anfallende wäßrige Phase gegebenenfalls nach Vereinigung mit wäßriger Phase aus der Nachextraktionsstufe (10) in der Produktextraktionsstufe (8) mit Anilin-haltigem, hydrophoben Lösungsmittel extrahiert,
g) die in der Produktextraktionsstufe (8) anfallende wäßrige Phase in zwei Teilströme (C) und (H) auftrennt, von denen einer an den Prozessanfang zurückgeführt und der andere der Nachextraktionsstufe (10) zugeführt wird,
h) die in der Produktextraktionsstufe (8) anfallende organische Phase in der Destillationsstufe (11) in ein aus Anilin-haltigem hydrophoben Lösungsmittel bestehendes Destillat und einen im wesentlichen aus Verfahrensprodukt bestehenden Destillationsrückstand auftrennt und
i) das in der Destillationsstufe (11) anfallende Destillat nach Zugabe von Frischanilin in der Produktextraktionsstufe (8) als Extraktionsmittel verwendet.

Ausgangsmaterialien für das erfindungsgemäße Verfahren sind Anilin und Formaldehyd oder formaldehydabspaltende Mittel. Der Formaldehyd wird vorzugsweise in Form einer wäßrigen Lösung mit einem Formaldehydgehalt von 20 bis 50 Gew.-% eingesetzt.

Bei den zum Einsatz gelangenden hydrophoben Lösungsmitteln handelt es sich um inerte Lösungsmittel des Siedepunktbereichs 30-250°C, vorzugsweise 80-200°C wie beispielsweise Chlorbenzol, Dichlorbenzole, Benzol, Toluol, Xylol, Dichlorethan, Chloroform oder Tetrachlorkohlenstoff. Vorzugsweise werden Xylole, d.h. technische Xylolgemische und insbesondere o-Xylol als hydrophobes Lösungsmittel verwendet.

Bei dem Säurekatalysator handelt es sich um wasserlösliche Säuren mit einem unter 2,5, vorzugsweise unter 1,5 liegendem pKa-Wert. Beispiele hierfür sind Salzsäure, Bromwasserstoffsäure, Schwefelsäure, Trifluoressigsäure, Methansulfonsäure oder Phosphorsäure. Bevorzugt einzusetzender Katalysator ist Salzsäure. Die genannten Säuren können auch im Gemisch mit sauren oder neutralen Salzen derartiger Säuren, wie z.B. den entsprechenden Ammoniumsalzen oder auch den entsprechenden Alkalisalzen eingesetzt werden; die Mitverwendung derartiger Salze ist jedoch weniger bevorzugt. Die genannten Säuren liegen in dem erfindungsgemäßen Kreislaufsystem in Form der entsprechenden Ammoniumsalze der im wäßrigen Kreislauf befindlichen Basen vor.

Das erfindungsgemäße Verfahren kann sowohl ein- als auch zweistufig und sowohl unter Vorschaltung einer Aminal-Vorstufe als auch ohne eine derartige Aminal-Vorstufe durchgeführt werden, wobei jedoch einschränkend hinzugefügt werden muß, daß bei einstufiger Reaktionsführung die Vorschaltung einer Aminal-Vorstufe auf jeden Fall angezeigt ist.

Unter "einstufiger Reaktionsführung" ist hierbei eine Verfahrensvariante zu verstehen, gemäß welcher das Aminal nach Hinzufügung des Säurekatalysators innerhalb eines kurzen Zeitraums von weniger als 10 Minuten, vorzugsweise von weniger als 5 Minuten auf eine erhöhte Temperatur von 60 bis 180, vorzugsweise 80 bis 150°C erhitzt wird, um dort in das Endprodukt umlagert zu werden, bzw. bei welcher das Aminal direkt mit der auf erhöhtem Temperaturniveau von 60-180°C, vorzugsweise 80 bis 150°C befindlichen und im Kreis geführten wäßrigen Katalysatorphase vermischt und das Gemisch dann gegebenenfalls bis zur angestrebten Endtemperatur erhitzt wird.

Unter "zweistufiger Reaktionsführung" ist eine Ausführungsform zu verstehen, gemäß welcher das Aminal nach Zugabe des Säurekatalysators oder das Reaktionsgemisch aus Anilin, Formaldehyd und Säurekatalysator zunächst während eines Zeitraums von 10 bis 90, vorzugweise 15 bis 60 Minuten in einer ersten Reaktionsstufe bei 0 bis 60°C, vorzugsweise 30 bis 60°C und anschließend während eines Zeitraums von 30 bis 180, vorzugsweise 30 bis 120 Minuten in einer zweiten Reaktionsstufe bei 60 bis 180°C, vorzugsweise 60 bis 150°C und insbesondere 95 bis 145°C gehalten wird. Bei dieser bevorzugten Verfahrensvariante einer mehrstufigen, vorzugsweise zweistufigen Reaktionsführung erfolgt in der ersten Reaktionsstufe vor allem eine Umlagerung des Aminals oder (im Falle eines Verzichts auf eine Aminalvorstufe) eine Kondensation der Ausgangsmaterialien zu N-Benzylanilin, welches in der zweiten Reaktionsstufe bei erhöhter Temperatur zum kernsubstituierten Endprodukt umgelagert wird. Gemäß einer besonderen Ausführungsform mit zweistufiger Reaktionsführung - ohne oder vorzugsweise mit Aminalvorstufe - wird die erste Reaktionsstufe nur mit einem Teilstrom der wäßrigen Katalysatorphase, im allgemeinen < 50 %, vorzugsweise < 15 %, eingeleitet. Im weiteren Verlauf der ersten Reaktionsstufe und vor Beendigung der letzten, d. h. im allgemeinen zweiten Reaktionsstufe erfolgt die Vervollständigung der Reaktion in Gegenwart der gesamten Katalysatorphase.

Das Verfahren kann sowohl kontinuierlich als auch diskontinuierlich durchgeführt werden; die angegebenen Zeiträume beziehen sich bei kontinuierlicher Fahrweise auf die mittlere Verweilzeit des Reaktionsgemisches in den einzelnen Stufen. Im Falle der Vorschaltung einer Aminal-Vorstufe beträgt die (mittlere) Verweilzeit der Ausgangsmaterialien in dieser Stufe im allgemeinen 10 bis 60, vorzugsweise 15 bis 60 Minuten. Die Temperatur in der Aminal-Vorstufe liegt im allgemeinen bei 20 bis 100°C, vorzugsweise 20 bis 60°C. Das Verfahren wird in allen Stufen vorzugsweise unter dem Eigendruck des Systems und vorzugsweise in einer Intergasatmosphäre (Stickstoff) durchgeführt.

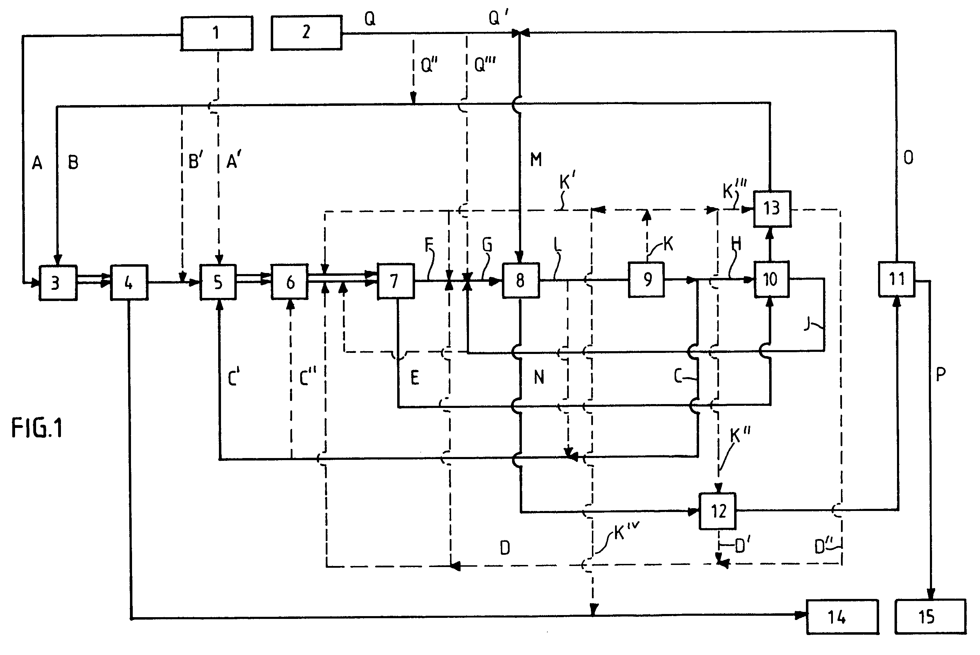

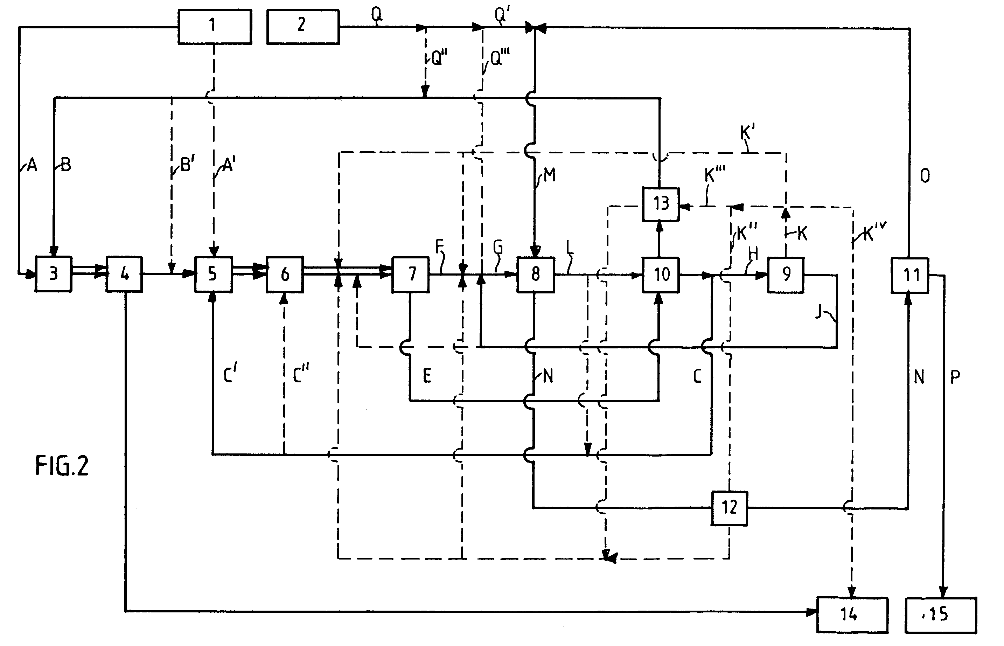

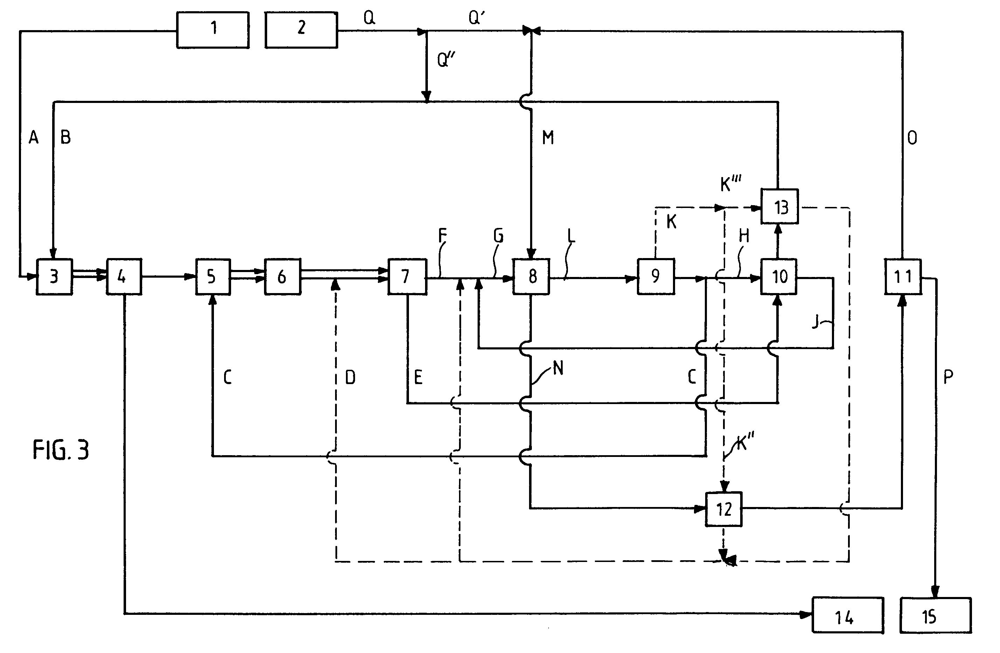

Die in den Figuren 1 - 3 dargestellten Fließdiagramme dienen der weiteren Erläuterung des erfindungsgemäßen Verfahrens. In diesen Abbildungen bedeuten:
(1) einen Tank für wäßrige Formaldehydlösung
(2) einen Tank für Anilin
(3) einen Kondensationsreaktor (Aminal-Vorstufe)
(4) einen Wasserabscheider
(5) die erste Reaktionsstufe
(6) die zweite Reaktionsstufe
(7) einen Phasenscheider
(8) die Produktextraktionsstufe
(9) einen Wasserverdampfer
(10) die Nachextraktionsstufe
(11) die Produktdestillationsstufe
(12) eine Waschstufe
(13) eine Waschstufe
(14) einen Tank für Abwasser
   und
(15) einen Tank für Verfahrensprodukt.

Die Bezugszeichen A bis Q bezeichnen die Mengenströme, auf die nachstehend und in den Beispielen Bezug genommen wird.

Im Falle der genannten einstufigen Reaktionsführung werden die Reaktionsstufe (5) und (6) zu einer einzigen Reaktionsstufe zusammengefaßt. Sowohl die erste als auch die zweite Reaktionsstufe können aus einem einzelnen als auch aus mehreren, in Serie geschalteten Reaktoren bestehen. Zur Aufrechterhaltung der genannten Verweilzeiten haben sich insbesondere in Serie geschaltete Rührkesselkaskaden und oder Kolonnenreaktoren bewährt.

Auch die Extraktionsstufe kann aus einem oder mehreren, in Serie geschalteten Extraktoren bestehen. Hier werden vorzugsweise die üblichen Gegenstromextraktionsvorrichtungen verwendet.

Die Destillationsstufe (11) besteht im einfachsten Fall aus einer Destillsationskolonne, die so ausgelegt ist, daß eine weitgehende Abtrennung von hydrophobem Lösungsmittel und Anilin vom Verfahrensprodukt möglich ist.

Als ein besonderer Vorteil des erfindungsgemäßen Verfahrens ist dabei der Umstand anzusehen, daß die Auftrennung von hydrophobem Lösungsmittel und Anilin nicht erforderlich ist, da der Anilingehalt im Destillat in der Regel nicht über dem für die Wiederverwendung erforderlichen Wert liegt, der vor der Wiederverwendung je nach Bedarf durch Zugabe von frischem Anilin eingestellt wird, und so der Einsatz energiesparender Mehrstufendestillationstechniken zur Bewältigung der Destillationsaufgabe ermöglicht wird.

Die Destillationsstufe (11) wird daher vorzugsweise mehrstufig betrieben, indem unter maximaler Ausnutzung der aufgewendeten Destillationsenergie in einer ersten Destillationsstufe eine Destillatfraktion erhalten wird, die das niedriger siedende hydrophobe Lösungsmittel in relativ angereicherter Form neben Anilin in relativ abgereicherter Form enthält und in einer letzten Destillationsstufe eine Destillatfraktion erhalten wird, die das hydrophobe Lösungsmittel in relativ abgereicherter und das Anilin in relativ angereicherter Form enthält. Bevorzugt ist eine Ausführungsform, bei der in einer letzten Destillationsstufe als Destillat Anilin nahezu frei von hydrophoben Lösungsmittel erhalten wird.

Das bei der Kondensation entstehende und das mit der wäßrigen Formaldehydlösung in das System eingebrachte Wasser muß an einer geeigneten Stelle zwecks Aufrechterhaltung eines konstanten Wasservolumens aus dem System entfernt werden. Bei Vorschaltung einer Aminalvorstufe (3) geschieht diese Wasserentfernung vorzugsweise im Wasserabscheider (4) bevor das Aminal mit dem Säurekatalysator zusammengebracht wird. In Abwesenheit einer Aminalvorstufe geschieht die Entfernung des Wassers vorzugsweise in einem Wasserverdampfer (9), der nach der Produktextraktionsstufe (8) angeordnet ist. Dieser Wasserverdampfer wird vorzugsweise nach dem Prinzip der Entspannungsverdampfung durch Anlegen von Vakuum betrieben.

Grundsätzlich ist es jedoch auch möglich, dem System Wasser an einer beliebigen anderen Stelle durch Destillation zu entnehmen.

Bei der Durchführung des erfindungsgemäßen Verfahrens sind mehrere Varianten möglich, die nachstehend im Detail beschrieben werden.

Gemäß einer 1. Variante des erfindungsgemäßen Verfahrens erfolgt die Einspeisung der wäßrigen Formaldehydlösung (A) in die Aminalstufe (3), in welcher die Umsetzung mit einem Gemisch (B) aus Anilin, gegebenenfalls Anilin-Formaldehyd-Kondensationsprodukten und hydrophobem Lösungsmittel, stattfindet. Bei dem Mengenstrom (B) handelt es sich im wesentlichen um die die Nachextraktionsstufe (10) bzw. die Waschstufe (13) verlassende organische Phase, der gegebenenfalls Anilin aus dem Vorratstank (2) zugesetzt wird.

Das Molverhältnis von Anilin zu Formaldehyd liegt im allgemeinen in der Aminalstufe zwischen 1,5:1 und 25:1, vorzugsweise bei 1,8:1 bis 10:1.

Das Gewichtsverhältnis von Arylamin ( = Anilin sowie gegebenenfalls vorliegende Anilin-Formaldehyd-Kondensationsprodukte) zu hydrophobem Lösungsmittel in (B) liegt im allgemeinen zwischen 1:4 und 3:1, vorzugsweise zwischen 1:2 und 2:1.

Die Umsetzung in der Aminalstufe (3) erfolgt innerhalb der obengenannten Temperaturbereiche.

Im Anschluß an (3) erfolgt in einem Scheider (4) die mechanische Abtrennung der wäßrigen Phase, welche von dem Kondensationswasser und dem Formalinwasser gebildet wird und daneben die wasserlöslichen Verunreinigungen des Formaldehyds und des Mengenstroms (B) enthält. Die Trennung erfolgt vorzugsweise unterhalb 60°C.

Die verbleibende organische Phase wird in den Reaktor (5) übergeführt und bei Temperaturen unterhalb von 60°C mit dem wäßrigen Mengenstrom (C') zusammengebracht.

Bei dem Mengenstrom (C') handelt es sich bei dieser ersten Variante des erfindungsgemäßen Verfahrens um die Gesamtmenge der zurückzuführenden Katalysatorphase (C). Der Gehalt an Anilin-Formaldehyd-Kondensaten in dieser Phase (C) liegt im allgemeinen unterhalb 5 Gew.-%, vorzugsweise unterhalb 2 Gew.-% bei einem Gesamtgehalt an Arylamin (inklusive Anilin) an dieser Stelle im allgemeinen von 30-70 Gew.-%, vorzugsweise 40-60 Gew.-%, und bei einem Protonierungsgrad von 25 bis 75, vorzugsweise 45 bis 70 %. Unter "Protonierungsgrad" ist hierbei und auch nachstehend der Prozentsatz der Aminstickstoffatome zu verstehen, die in Form von Ammoniumgruppen, d.h. "protoniert" vorliegen.

Das Gewichtsverhältnis zwischen organischer Phase aus (4) und Katalysatorphase (C) liegt im allgemeinen zwischen 100:1 und 1:10, vorzugsweise zwischen 4:1 und 1:3.

Bei der ersten, kontinuierlich betriebenen Variante des erfindungsgemäßen Verfahrens stellt der Reaktor (5) die bereits obengenannte "erste Reaktionsstufe" dar, die unter den obengenannten Bedingungen bezüglich der Temperatur und der Reaktionszeit betrieben wird. Im allgemeinen handelt es sich um eine mehrstufige Rührkesselkaskade oder einen ein- oder mehrstufigen Kolonnenreaktor, in welchem vorzugsweise ein Temperaturprofil von ca. 20°C am Beginn ansteigend bis auf 60°C am Ende durchlaufen wird.

Aus der ersten Reaktionsstufe (5) wird das zweiphasige Reaktionsgemisch in die zweite Reaktionsstufe (6) überführt, die ebenfalls aus einer mehrstufigen Rührkesselkaskade oder einem ein- oder mehrstufigen Kolonnenreaktor besteht. Diese zweite Reaktionsstufe wird ebenfalls unter den bereits obengenannten Bedingungen bezüglich der Reaktionstemperatur und der mittleren Verweilzeit betrieben. Vorzugsweise durchläuft das zweiphasige Reaktionsgemisch in der Reaktionsstufe (6) ein Temperaturprofil beginnend bei 60°C und endend bei einer Temperatur zwischen 90 und 150°C, vorzugsweise zwischen 95 und 140°C. Im Falle dieser bevorzugten Temperaturführung sind in der Reaktionsstufe (6) im allgemeinen Verweilzeiten von bis zu 60 Minuten ausreichend.

Das die zweite Reaktionsstufe (6) verlassende zweiphasige Reaktionsgemisch wird anschließend im Phasenscheider (7), vorzugsweise bei Temperaturen zwischen 80 und 110°C in eine organische Phase (E) und eine wäßrige Phase (F) zerlegt, die mit dem Strom (J) zur wäßrigen Phase (G) vereinigt und der Produktextraktionsstufe (8) zugeführt wird.

Aus der wäßrigen Phase (G) werden in dem vorzugsweise mehrstufig wirkenden Extraktor (8), der vorzugsweise bei Temperaturen von 80 bis 110°C betrieben wird, die Verfahrensprodukte im Austausch gegen Anilin extrahiert und in eine organische Lösung (N) überführt.

Als Extraktionsmittel (M) dient ein Gemisch aus hydrophobem Lösungsmittel und Anilin. Das Gewichtsverhältnis Anilin : Lösungsmittel liegt im allgemeinen zwischen 0,5:1 und 3:1, vorzugsweise 1:1 und 2:1.

Das Gewichtsverhältnis Extraktionsmittel (M) : wäßrige Phase (G) liegt im allgemeinen zwischen 0,5:1 und 3:1, vorzugsweise bei 0,7:1 bis 2:1.

Die organische Phase (N) wird, gegebenenfalls nach Durchlaufen der Katalysatorwaschstufe (12), in der gegebenenfalls Katalysatorspuren entfernt werden, in die Destillationsstufe (11) überführt.

In der Destillationsstufe (11) erfolgt die destillative Abtrennung eines Destillationsrückstandes (P), der das Verfahrensprodukt darstellt und in Tank (15) gesammelt wird.

Die Destillationsstufe (11) kann beispielsweise aus einem einstufigen Verdampfer bestehen, der neben dem Destillationsrückstand (P) ein Destillat (O) liefert.

Das Destillat (O) enthält neben Anilin das gesamte hydrophobe Lösungsmittel aus (N) und wird gegebenenfalls nach Zusatz von Frischanilin (Q') als Extraktionsmittel (M) verwendet.

Die Produktextraktionsstufe (8) verlassende wäßrige Phase (L) enthält nur noch sehr geringe Anteile von unter 5 Gew.-%, vorzugsweise von unter 2 Gew.-%, an Verfahrensprodukten (Anilin/Formaldehyd-Kondensate) und wird in zwei Teilströme C und H aufgeteilt, wobei das Gewichtsverhältnis C : H im allgemeinen 1:100 bis 10:1, vorzugsweise 1:3 bis 3:1, liegt.

Der Teilstrom C wird als wäßrige Katalysatorlösung in die erste Reaktionsstufe (5) zurückgeführt (C = C').

Mit dem Teilstrom H wird in der mehrstufig arbeitenden Nachextraktionsstufe (10), die im allgemeinen bei Temperaturen von 40°C - 110°C betrieben wird, die organische Phase (E) aus dem Scheider (7) extrahiert, wobei in (E) enthaltene Verfahrensprodukte weitgehend gegen Anilin ausgetauscht und in die wäßrige Phase (J) überführt werden, so daß bei der 1. Variante des Verfahrens eine organische Phase (B) resultiert, die an Kondensationsprodukten verarmt ist.

Der Restgehalt an Verfahrensprodukten in (B) beträgt in der Regel <10 Gew.-%, vorzugsweise <5 Gew.-%. In diesem Rest an Kondensationsprodukten liegt u.a. das 2,2'-Isomere des Diaminodiphenylmethans in relativ angereicherter Form vor.

Ebenso verbleiben die gegebenenfalls am Ende der Umlagerungsreaktion in (6) im Reaktionsgemisch noch vorliegenden Kondensationszwischenprodukte zunächst bei der Phasentrennung im Scheider (7) in der vorliegenden Ausführungsform nahezu quantitativ in der organischen Phase (E), in der sie sich beim Durchlaufen der Nachextraktionsstufe (10) in der Restfraktion aufkonzentrieren und anschließend mit dem Mengenstrom (B) rezykliert werden.

Die in der Nachextraktionsstufe (10) anfallende organische Phase (B) wird in die Aminalstufe (3) zurückgeführt, nachdem gegebenenfalls Anilin (Q'') beispielsweise aus Vorratstank (2) zugesetzt wurde.

Die in der Nachextraktionsstufe (10) anfallende wäßrige Phase (J) wird in den Reaktionsprozess zwischen der letzten Reaktionsstufe (6) und der Hauptextraktionsstufe (8), vorzugsweise zwischen (7) und (8), zurückgeführt, wo sie mit der wäßrigen Phase (F) aus dem Phasenscheider (7) zur wäßrigen Phase (G) vereinigt wird.

Vorzugsweise wird bei der Durchführung der 1. Variante des erfindungsgemäßen Verfahrens zwischen der Produktextraktionsstufe (8) und der Nachextraktionsstufe (10) der bereits früher erwähnte Wasserverdampfer angeordnet, wobei die Aufteilung des Mengenstroms (L) in die Mengenströme (C) und (H) vor oder hinter dem Verdampfer erfolgen kann. In dem Wasserverdampfer (9) wird der betreffenden wäßrigen Lösung (L bzw. H) eine Wassermenge (K) entzogen, die im allgemeinen bis zu 80 Gew.-%, vorzugsweise jedoch weniger als 50 Gew.-%, der Wassermenge der in den Verdampfer eingebrachten wäßrigen Phase ausmacht. Die Wassermenge (K) wird vorzugsweise zwischen der zweiten Reaktionsstufe (6) und der Produktextraktionsstufe (8) in das Reaktionsgemisch zurückgeführt (K'), gegebenenfalls vorher jedoch zumindest teilweise zum Waschen der die Produktextraktionsstufe (8) verlassenden organischen Phase zwecks Entfernung von Säurespuren (K'') und/oder zum Waschen der an den Prozessanfang zurückzuführenden organischen Phase (B) (K''') verwendet, wobei in den betreffenden Waschstufen (12) bzw. (13) die wäßrigen Phasen (D') bzw. (D'') resultieren.

Bei dieser Arbeitsweise (Entfernung von Wasser in (9) und Rückführung) wird die Umlagerung in den Reaktoren (5) und (6) bei einem geringeren Wassergehalt der wäßrigen Phase durchgeführt als die Extraktion in der Extraktionsstufe (8).

Die 2. Variante des erfindungsgemäßen Verfahrens besteht darin, auf die Aminalstufe (3) teilweise oder vollständig zu verzichten. In der Praxis bedeutet dies, daß eine Teilmenge des in der Reaktion eingesetzten Gemischs aus Arylamin und hydrophobem Lösungsmittel (B') und/oder eine Teilmenge des eingesetzten wäßrigen Formaldehyds (A') nicht in die Aminalstufe (3) sondern vor bzw. in die erste Reaktionsstufe (5) geleitet werden. Im Extremfall (völliger Verzicht auf eine Aminalvorstufe) kann auch die Gesamtmenge des Gemischs aus Anilin und hydrophobem Lösungsmittel und die Gesamtmenge des wäßrigen Formaldehyds direkt zur ersten Reaktionsstufe (5) geleitet werden. Bei Verzicht auf eine Aminalvorstufe muß jedoch, wie oben ausgeführt, die Reaktion auf jeden Fall zweistufig unter Verwendung der Reaktionsstufen (5) und (6) durchgeführt werden. Bei einer solchen Arbeitsweise wird naturgemäß das eingebrachte und durch die Kondensationentstehende Wasser nur teilweise oder überhaupt nicht über den Phasenscheider (4) entfernt. Die Entfernung dieses Wassers geschieht dann beispielsweise aus dem Destillat aus (10) als Mengenstrom K ^{IV}, der direkt in den Abwassertank (14) geleitet wird.

Gemäß einer 3. Variante können die Reaktionsstufen (5) und (6) zu einer einzigen Reaktionsstufe zusammengefaßt werden, die dann unter den obengenannten Bedingungen bezüglich der Reaktionstemperatur und der Reaktionszeit betrieben wird. Im Falle einer einstufigen Reaktionsführung ist jedoch auf jeden Fall eine vorgeschaltete Aminalstufe (3) erforderlich.

Gemäß einer 4., mit der 1. und 2. Variante kombinierbaren Variante erfolgt in der ersten Reaktionsstufe die Umsetzung in Gegenwart von lediglich einem Teil (C') der zurückgeführten Katalysatorlösung und unter Zugabe der verbleibenden Menge an Katalysatorlösung (C'', C'''und c^{IV}) hinter der ersten Reaktionsstufe (5) und vor der Produktextraktionsstufe (8). Vorzugsweise wird gemäß dieser 4. Variante so gearbeitet, daß man den Katalysatorstrom (C) in zwei Teilströme (C') und (C'') auftrennt und den ersten Teilstrom (C') in die erste Reaktionsstufe (5) und den zweiten Teilstrom (C'') im weiteren Verlauf der ersten Reaktionsstufe (5) und vor bzw. in der zweiten Reaktionsstufe (6) in das Reaktionsgemisch einleitet. Das Gewichtsverhältnis zwischen organischer Phase in (5) und zunächst zugeführter wäßriger Phase (C') z.B. im ersten Rührkessel von (5) liegt zwischen 1:1 und 100:1, vorzugsweise zwischen 3:1 und 30:1.

Gemeinsam ist den beschriebenen Varianten des erfindungsgemäßen Verfahrens, daß es in der Nachextraktionsstufe (10) vorzugsweise zu einer weitgehend selektiven Auftrennung der in (E) enthaltenden Reaktionsprodukte, d.h. der am Ende des eigentlichen Reaktionsteils (6) vorliegenden und in Scheider (7) mit (E) abgetrennten Fraktion der Reaktionsprodukte kommt in:
1. eine in der die Nachextraktionsstufe (10) verlassenden organischen Phase verbleibende Restfraktion. In dieser Restfraktion sind die bereits in (E) gegenüber (F) angereicherten Bestandteile wie Kondensationszwischenprodukte vom N-Aminobenzylamintyp, gegebenenfalls vom Aminaltyp, Kondensationsendprodukte wie 2,2'-Diaminodiphenylmethan, gegebenenfalls 2,4'-Diaminodiphenylmethan und gegebenenfalls andere nicht näher spezifizierte Zwischen-, End- und/oder Nebenprodukte, aufkonzentriert und werden mit der organischen Phase (B) an den Anfang des Reaktionsgeschehens rezykliert,
2. eine Produktfraktion die sich in der wäßrigen Phase (J) befindet und in der die unter 1. aufgeführten Produktkomponenten relativ abgereichert sind. Die in dieser wäßrigen Phase (J) enthaltenen Produktanteile gelangen verfahrensgemäß indirekt über die Verfahrensstufe (7) oder vorzugsweise direkt durch Vereinigung von (J) mit der wäßrigen Phase (F) zum Mengenstrom (G) in die Extraktionsstufe (8) und damit in das isolierte Verfahrensendprodukt.
   Dadurch kann in der Regel in den isolierten Verfahrensendprodukten auch bei höheren Gehalten des 2,4'-Isomeren z.B. >10 % vom Gesamtgehalt an Diaminodiphenylmethan, das Verhältnis von 2,2'-Isomeren zu 2,4'-Isomeren unter 1:20 gehalten werden. Im allgemeinen beträgt der Anteil an 2,2'-Diaminodiphenylmethan im isolierten Endprodukt weniger als 50 % des Gehaltes vergleichbarer jedoch ohne die erfindungsgemäße Durchführung hergestellter Produkte.

Grundsätzlich sollte bei allen Varianten die Destillationsstufe (11) zugeführte organische Phase (8) praktisch frei von Säurespuren sein. Dies kann durch intensives Waschen in der Waschstufe (12) und/oder durch Neutralisation in einer Neutralisationsstufe (nicht gezeichnet) erfolgen.

Die nachfolgenden Beispiele dienen der weiteren Erläuterung des erfindungsgemäßen Verfahrens.

### Beispiel 1

In einem aus zwei hintereinander geschalteten Rührkesseln bestehenden Reaktor (3) wird 30 %ige wäßrige Formalinlösung (Mengenstrom A) mit einem Anilin-Xylolgemisch, welches noch Polyarylaminanteile enthält (Mengenstrom B) bei 40°C zur Reaktion gebracht.
- (A): 0,62 kg/h Formaldehyd
1,45 kg/h Wasser
- (B): 0,06 kg/h Polyarylamin
4,07 kg/h Anilin
3,55 kg/h ortho-Xylol
In dem anschließenden Scheider (4) wird die untere, wäßrige Phase als Abwasser abgetrennt und in dem Abwassertank (14) gesammelt.

Die obere organische Phase wird in einem zweiten, aus drei Rührkesseln bestehenden Reaktor (5) übergeführt und dort mit dem Mengenstrom (C), der den sauren Katalysator enthält, vermischt.
- (C): 0,04 kg/h Polyarylamin
1,69 kg/h Anilin
0,40 kg/h Chlorwasserstoff
2,38 kg/h Wasser
Die gemessenen und geregelten Temperaturen in den drei Kesseln des Reaktors (5) betragen 30°C, 40°C und 60°C.

In einem weiteren, ebenfalls aus drei Rührkesseln bestehenden Reaktor (6) werden die Temperaturen 100°C, 135°C und 140°C durchlaufen, die durch Aufheizen unter dem sich einstellenden Eigendruck des Systems eingestellt werden.

Nach dem Abkühlen des Reaktionsgemisches auf 95°C und Entspannen auf Normaldruck und nach Zugabe des HCl-Waschwassers aus der Extraktionsstufe (12) und (13) werden im Phasenscheider (7) die organische Phase (Mengenstrom E) und die wäßrige Phase (Mengenstrom F) voneinander getrennt.
- (E): 1,81 kg/h Polyarylamin
1,61 kg/h Anilin
3,55 kg/h ortho-Xylol
- (F): 1,89 kg/h Polyarylamin
0,81 kg/h Anilin
0,40 kg/h Chlorwasserstoffsäure
3,88 kg/h Wasser
Die wäßrige Phase (F) wird anschließend zusammen mit der wäßrigen Phase (J) aus der Nachextraktionsstufe (10) in der Extraktionskolonne (8) im Gegenstrom mit einem eingestellten Anilin-Xylolgemisch (Mengenstrom M) kontinuierlich extrahiert.
- (M): 8,45 kg/h Anilin
7,05 kg/h ortho-Xylol

und geht dabei über in die an Polyarylamin verarmte wäßrige Phase Mengenstrom (L).
- (L): 0,08 kg/h Polyarylamin
3,28 kg/h Anilin
0,77 kg/h Chlorwasserstoffsäure
7,41 kg/h Wasser
Der Teilstrom (L) wird in der Destillationsstufe (9) aufkonzentriert unter Entnahme von Destillat als Mengenstrom (K) und anschließend aufgeteilt in zwei Teilströme (C) und (H).
- (C): 4,50 kg/h
- (H): 4,24 kg/h
Der Teilstrom (H) wird in einer weiteren Extraktionskolonne (10) zur Gegenstromextraktion der in (7) abgetrennten organischen Phase (E) eingesetzt.

Die in (10) resultierende, gegenüber dem eingesetzten wäßrigen Teilstrom (H) an Polyarylamin angereicherte wäßrige Phase (Mengenstrom (J)) wird mit der wäßrigen Phase (F) aus Scheider (7) vereinigt und gemeinsam als Mengenstrom (G) mit dieser in (8) extrahiert.
- (J): 1,78 kg/h Polyarylamin
1,31 kg/h Anilin
0,31 kg/h Chlorwasserstoffsäure
2,24 kg/h Wasser
Die in der Extraktionsstufe (10) resultierende an Polyarylamin gegenüber (E) verarmte organische Phase wird nach Durchlaufen der Waschstufe (13) und Zusatz von Anilin (Q'') als Mengenstrom (B) in den Reaktor (3) zurückgeführt.

Die in der Produktextraktion (8) anfallende das Reaktionsprodukt enthaltende organische Phase (Mengenstrom N) wird in einer weiteren 3-5 stufig wirkenden Extraktionskolonne (12) mit der Hauptmenge des im wesentlichen aus Wasser bestehenden Destillats der Destillationsstufe (9) (Mengenstrom K^{II}) extrahiert.
- (N): 3,59 kg/h Polyarylamin
7,30 kg/h Anilin
7,05 kg/h ortho-Xylol
- (K): ca. 2,8 kg/h Wasser
In der Waschstufe (12) wird der HCl-Gehalt des Mengenstroms (N), der bei ca. 0,2 Gew.-% liegt reduziert auf <0,01 Gew.-%.

Das HCl-haltige Waschwasser (ca. 2,8 kg/h) wird als Mengenstrom (D) in das Reaktionsgemisch recyclisiert.

Die die Waschkolonne (12) verlassende organische Phase wird nach Entfernung restlicher Säurespuren durch Neutralisation mit überschüssiger Natronlauge und Abtrennung des gebildeten Kochsalzes und der unverbrauchten Natronlauge in einer Destillationsstufe (11) aufgetrennt in ein Destillat (Mengenstrom O) und einen Destillationsrückstand (Mengenstrom P).
- (O): 7,30 kg/h Anilin
7,05 kg/h ortho-Xylol
- (P): ca. 3,5 kg/h Polyarylamin
Durch Zugabe von frischem Anilin (Q') aus dem Vorratsbehälter (2) zum Destillat (O) wird das in (8) benötigte Extraktionsmittel nach Menge und Zusammensetzung eingestellt (Mengenstrom M).

Der Destillationsrückstand (Mengenstrom P) der Destillationsstufe (11) hat folgende Zusammensetzung:
0,4 Gew.-% 2,2'-Diaminodiphenylmethan
9,7 Gew.-% 2,4'-Diaminodiphenylmethan
59,7 Gew.-% 4,4'-Diaminodiphenylmethan
0,2 Gew.-% N-substituierte Diaminodiphenylmethane
19,2 Gew.-% Triamine
6,0 Gew.-% Tetramine
ca. 4,8 Gew.-% hoher als vierfunktionelle Polyarylamine

### Beispiel 2

In einem aus zwei hintereinander geschalteten Rührkesseln bestehenden Reaktor (3) wird 30 %ige wäßrige Formalinlösung (Mengenstrom A) mit einem Anilin-Xylolgemisch, welches noch Polyarylaminanteile enthält (Mengenstrom B) bei 40°C zur Reaktion gebracht.
- (A): 0,62 kg/h Formaldehyd
1,45 kg/h Wasser
- (B): 0,22 kg/h Polyarylamin
3,82 kg/h Anilin
3,26 kg/h ortho-Xylol
In dem anschließenden Scheider (4) wird die untere, wäßrige Phase als Abwasser abgetrennt und in dem Abwassertank (14) gesammelt.

Die obere organische Phase wird in einem zweiten, aus drei Rührkesseln bestehenden Reaktor (5) übergeführt und dort mit dem Mengenstrom (C), der den sauren Katalysator enthält, vermischt.
- (C): 0,07 kg/h Polyarylamin
2,21 kg/h Anilin
0,54 kg/h Chlorwasserstoff
3,18 kg/h Wasser
Die gemessenen und geregelten Temperaturen in den drei Kesseln des Reaktors (5) betragen 30°C, 40°C und 60°C.

In einem weiteren, ebenfalls aus drei Rührkesseln bestehenden Reaktor (6) werden die Temperaturen 100°C, 135°C und 140°C durchlaufen, die durch Aufheizen unter dem sich einstellenden Eigendruck des Systems eingestellt werden.

Nach dem Abkühlen des Reaktionsgemisches auf 95°C und Entspannen auf Normaldruck und nach Zugabe des HCl-Waschwassers aus der Extraktionsstufe (12) und (13) werden im Phasenscheider (7) die organische Phase (Mengenstrom E) und die wäßrige Phase (Mengenstrom F) voneinander getrennt.
- (E): 1,48 kg/h Polyarylamin
1,37 kg/h Anilin
3,26 kg/h ortho-Xylol
- (F): 2,40 kg/h Polyarylamin
1,32 kg/h Anilin
0,54 kg/h Chlorwasserstoffsäure
4,98 kg/h Wasser
Die wäßrige Phase (F) wird anschließend zusammen mit der wäßrigen Phase (J) aus der Nachextraktionsstufe (10) in der Extraktionskolonne (8) im Gegenstrom mit einem eingestellten Anilin-Xylolgemisch (Mengenstrom M) kontinuierlich extrahiert.
- (M): 8,9 kg/h Anilin
8,1 kg/h ortho-Xylol

und geht dabei über in die an Polyarylamin verarmte wäßrige Phase Mengenstrom (L).
- (L): 0,12 kg/h Polyarylamin
3,68 kg/h Anilin
0,90 kg/h Chlorwasserstoff
8,30 kg/h Wasser
Der Mengenstrom (L) wird aufgeteilt in zwei Teilströme (C) und (H).
- (C): 5,20 kg/h
- (H): 7,80 kg/h
Der Teilstrom (C) wird in der Destillationsstufe (9) aufkonzentriert unter Entnahme von Destillat (Mengenstrom K) und anschließend als Mengenstrom (C) in den Reaktor (5) zurückgeführt.

Der Teilstrom (H) wird in einer weiteren Extraktionskolonne (10) zur Gegenstromextraktion der in (7) abgetrennten organischen Phase (6) eingesetzt.

Die in (10) resultierende, gegenüber dem eingesetzten wäßrigen Teilstrom (H) an Polyarylamin angereicherte wäßrige Phase (Mengenstrom (J) wird mit der wäßrigen Phase (F) aus Scheider (7) vereinigt und gemeinsam mit dieser in (8) extrahiert.
- (J): 1,30 kg/h Polyarylamin
1,12 kg/h Anilin
0,36 kg/h Chlorwasserstoff
3,32 kg/h Wasser
Die in der Extraktionsstufe (10) resultierende an Polyarylamin gegenüber (E) verarmte organische Phase wird nach Durchlaufen der Waschstufe (13) als Mengenstrom (B) in den Reaktor (3) zurückgeführt.

Die in der Produktextraktion (8) anfallende das Reaktionsprodukt enthaltende organische Phase (Mengenstrom N) wird in einer weiteren Extraktionskolonne (12) mit dem im wesentlichen aus Wasser bestehenden Destillat der Destillationsstufe (9) (Mengenstrom K) extrahiert.
- (N): 3,59 kg/h Polyarylamin
7,66 kg/h Anilin
8,10 kg/h ortho-Xylol
- (K''): 1,50 kg/h Wasser
In der Waschstufe (12) wird der HCl-Gehalt des Mengenstroms (N), der bei ca. <0,2 Gew.-% liegt reduziert auf <0,01 Gew.-%.

Die HCl-haltigen Waschwässer (ca. 1,8 kg) aus (12) und (13) werden als Mengenstrom (D) in das Reaktionsgemisch recyclisiert.

Die die Waschkolonne (12) verlassende organische Phase wird nach Entfernung restlicher Säurespuren durch Neutralisation mit überschüssiger Natronlauge und Abtrennung des gebildeten Kochsalzes und der unverbrauchten Natronlauge in einer Destillationsstufe (11) aufgetrennt in ein Destillat (Mengenstrom O) und einen Destillationsrückstand (Mengenstrom P).
- (O): 7,66 kg/h Anilin
8,10 kg/h ortho-Xylol
- (P): ca. 3,5 kg/h Polyarylamin
Durch Zugabe von frischem Anilin (Q) aus dem Vorratsbehälter (2) zum Destillat (O) wird das in (8) benötigte Extraktionsmittel nach Menge und Zusammensetzung eingestellt (Mengenstrom M).

Der Destillationsrückstand (Mengenstrom P) der Destillationsstufe (11) hat folgende Zusammensetzung:
<0,2 Gew.-% 2,2'-Diaminodiphenylmethan
6,7 Gew.-% 2,4'-Diaminodiphenylmethan
60,3 Gew.-% 4,4'-Diaminodiphenylmethan
<0,2 Gew.-% N-substituierte Diaminodiphenylmethane
20,2 Gew.-% Triamine
7,5 Gew.-% Tetramine
ca. 4,9 Gew.-% höher als vierfunktionelle Polyarylamine

## Patentansprüche

1. Verfahren zur Herstellung von mehrkernigen aromatischen Polyaminen durch Umsetzung von Anilin mit Formaldehyd in Gegenwart von Wasser und sauren Katalysatoren in einer ein- oder zweistufigen Reaktion innerhalb des Temperaturbereichs von 0 bis 180°C, gegebenenfalls unter Vorschaltung einer Aminal-Vorstufe, in welcher die Bildung von N,N'-disubstituiertem Aminal stattfindet, welches dann ein- oder mehrstufig in Gegenwart von Säurekatalysator innerhalb des Temperaturbereichs von 0 bis 180°C in das gewünschte Endprodukt überführt wird, Aufarbeitung des resultierenden Reaktionsgemischs durch Extraktion mit einem Anilin-haltigen hydrophoben Lösungsmittel in einer Produktextraktionsstufe (8) destillative Auftrennung der hierbei anfallenden organischen Phase in (i) ein aus Anilinhaltigem Lösungsmittel bestehendes Destillat, welches, gegebenenfalls nach Zugabe von Frischanilin erneut bei der Extraktion eingesetzt wird und (ii) einen im wesentlichen aus Verfahrensprodukt bestehenden Destillationsrückstand und Rückführung von bei der Extraktion anfallender, den Säurekatalysator enthaltender wäßriger Phase unter Wiederverwendung des in ihr enthaltenen Katalysators, unter Entfernung des bei der Kondensationsreaktion entstehenden Kondensationswassers und des in das System mit der wäßrigen Lösung des Formaldehyds eingebrachten Wassers in einem der Aminalvorstufe nachgeschalteten und der ersten Reaktionsstufe vorgeschalteten Wasserabscheider und/oder in einem der Extraktionsstufe nachgeschalteten Verdampfer, dadurch gekennzeichnet, daß man
a) den in Form einer wäßrigen Lösung zum Einsatz gelangenden Formaldehyd in einer Aminal-Vorstufe (3) mit einer aus Anilin, gegebenenfalls Anilin-Formaldehyd-Kondensationsprodukten und aus hydrophobem Lösungsmittel bestehenden organischen Phase und/oder in der ersten Reaktionsstufe (5) mit einer aus Anilin, gegebenenfalls Anilin-Formaldehyd-Kondensationsprodukten und aus hydrophobem Lösungsmittel bestehenden organischen Phase und recyclisierter, den Katalysator in Form von Aminsalzen enthaltender wäßriger Phase durch Vermischen zur Reaktion bringt,
b) das so erhaltene zweiphasige Reaktionsgemisch nach Beendigung der Reaktion in einem der Produktextraktionsstufe (8) vorgeschalteten Phasenscheider (7) in eine wäßrige Phase und eine organische Phase auftrennt,
c) die im Phasenscheider (7)anfallende organische Phase in einer der Produktextraktionsstufe (8) nachgeschalteten Nachextraktionsstufe (10) mit einem Teil oder gegebenenfalls mit der Gesamtmenge der in der Produktextraktionsstufe (8) anfallenden, weitgehend vom Reaktionsprodukt befreiten wäßrigen Phase extrahiert,
d) die in der Nachextraktionsstufe (10) anfallende mit Reaktionsprodukt der im Phasenscheider (7) anfallenden organischen Phase angereicherte wäßrige Phase vollständig oder zumindest teilweise in den Reaktionsprozess nach beendeter Umlagerung (6) und vor der Hauptextraktionsstufe (8) zurückführt und den gegebenenfalls verbleibenden Rest der wäßrigen Phase vor beendeter Umlagerung (6) zurückführt,
e) die in der Nachextraktionsstufe (10) anfallende, aus Anilin, gegebenenfalls Anilin-Formaldehyd-Kondensationsprodukten und aus hydrophobem Lösungsmittel bestehende organische Phase an den Prozessanfang zurückführt und gemäß a) umsetzt,
f) die im Phasenscheider (7) anfallende wäßrige Phase gegebenenfalls nach Vereinigung mit wäßriger Phase aus der Nachextraktionsstufe (10) in der Produktextraktionsstufe (8) mit Anilin-haltigem, hydrophoben Lösungsmittel extrahiert,
g) die in der Produktextraktionsstufe (8) anfallende wäßrige Phase in zwei Teilströme (C) und (H) auftrennt, von denen einer an den Prozessanfang zurückgeführt und der andere der Nachextraktionsstufe (10) zugeführt wird oder die in der Produktextraktionsstufe (8) anfallende wäßrige Phase in die Nachextraktionsstufe (10) führt und danach in zwei Teilströme (C) und (H) auftrennt, von denen einer an den Prozeßanfang zurückgeführt und der andere nach beendeter Umlagerung (6) und vor der Hauptextraktionsstufe (8) zurückgeführt wird,
h) die in der Produktextraktionsstufe (8) anfallende organische Phase in der Destillationsstufe (11) in ein aus Anilin-haltigem hydrophoben Lösungsmittel bestehendes Destillat und einen im wesentlichen aus Verfahrensprodukt bestehenden Destillationsrückstand auftrennt und
i) das in der Destillationsstufe (11) anfallende Destillat nach Zugabe von Frischanilin in der Produktextraktionsstufe (8) als Extraktionsmittel verwendet.

2. Verfahren gemäß Anspruch 1, dadurch gekennzeichnet, daß man der die Produktextraktionsstufe (8) verlassenden wäßrigen Phase vor oder nach ihrer Auftrennung in zwei Teilströme in einer Destillationsstufe (9) destillativ eine Wassermenge entzieht, die über die Wassermenge hinausgeht, die zur Aufrechterhaltung eines konstanten Wasservolumens im Kreislauf notwendig ist und diese über die zur Konstanthaltung des Wasservolumens hinausgehende Wassermenge an einer beliebigen Stelle hinter der letzten Reaktionsstufe (6) und vor der Extraktionsstufe (8) in den Kreislauf zurückführt.

3. Verfahren gemäß Anspruch 2, dadurch gekennzeichnet, daß man das in der Destillationsstufe (9) als Destillat anfallende Wasser vor seiner Rückführung zumindest teilweise zum Waschen der die Produktextraktionsstufe (8) verlassenden organischen Phase und/oder der die Nachextraktionsstufe (10) verlassenden organischen Phase verwendet.

4. Verfahren gemäß Anspruch 1 bis 3, dadurch gekennzeichnet, daß man die an den Prozessanfang zurückzuführende Katalysatorlösung (C) in zwei oder mehrere Teilströme aufteilt und den ersten Teilstrom in die erste Reaktionsstufe (5) und die anderen Teilströme im weiteren Verlauf der ersten Reaktionsstufe (5) und vor der Produktextraktionsstufe (8) in das Reaktionsgemisch zurückleitet.

## Claims

1. A process for the production of polynuclear aromatic polyamines by reaction of aniline with formaldehyde in the presence of water and acidic catalysts in one or two stages at a temperature in the range from 0 to 180°C, optionally with inclusion of a preliminary aminal stage in which N,N'-disubstituted aminal is formed and is then converted into the desired end product in one or more stages in the presence of acid catalyst at a temperature in the range from 0 to 180°C, working up of the resulting reaction mixture by extraction with an aniline-containing hydrophobic solvent in a product extraction stage (8), separation of the organic phase accumulating by distillation into (i) a distillate consisting of aniline-containing solvent, which is reused in the extraction stage, optionally after addition of fresh aniline, and (ii) a distillation residue consisting essentially of end product and recycling of aqueous phase accumulating during extraction and containing the acid catalyst with reuse of the catalyst present therein and with removal of the water of condensation formed in the condensation reaction and the water introduced into the system with the aqueous formaldehyde solution in a water separator following the preliminary aminal stage and preceding the first reaction stage and/or in an evaporator following the extraction stage, characterized in that
a) the formaldehyde used in the form of an aqueous solution is reacted by mixing in a preliminary aminal stage (3) with an organic phase consisting of aniline, optionally aniline/formaldehyde condensates and hydrophobic solvent and/or in a first reaction stage (5) with an organic phase consisting of aniline, optionally aniline/formaldehyde condensates and hydrophobic solvent and recycled aqueous phase containing the catalyst in the form of amine salts,
b) the two-phase reaction mixture obtained is separated on completion of the reaction into an aqueous phase and an organic phase in a phase separator (7) preceding the product extraction stage (8),
c) the organic phase accumulating in the phase separator (7) is extracted in a post-extraction stage (10) following the product extraction stage (8) with part of the aqueous phase substantially freed from reaction product which accumulates in the product extraction stage (8),
d) the aqueous phase accumulating in the post-extraction stage (10), which is enriched with reaction product of the organic phase accumulating in the phase separator (7), is completely or at least partly recycled to the reaction before the completion of rearrangement (6) and before the main extraction stage (8) and any aqueous phase remaining is recycled before the completion of rearrangement (6),
e) the organic phase consisting of aniline, optionally aniline/formaldehyde condensates and hydrophobic solvent, which accumulates in the post-extraction stage (10), is returned to the beginning of the process and reacted in accordance with a),
f) optionally after combination with aqueous phase from the post-extraction stage (10), the aqueous phase accumulating in the phase separator (7) is extracted with aniline-containing hydrophobic solvent in the product extraction stage (8),
g) the aqueous phase accumulating in the product extraction stage (8) is divided into two partial streams (C) and (H) of which one is returned to the beginning of the process while the other is delivered to the post extraction stage (10) or the aqueous phase accumulating in the product extraction stage (8) is delivered to the post extraction stage (10) is then divided into two partial streams (C) and (H) of which one is returned to the beginning of the process while the other is returned on completion of rearrangement (6) and before the main extraction stage (8),
h) the organic phase accumulating in the product extraction stage (8) is separated in the distillation stage (11) into a distillate consisting of aniline-containing hydrophobic solvent and a distillation residue consisting essentially of end product and
i) the distillate accumulating in the distillation stage (11) is used as extractant in the product extraction stage (8) after addition of fresh aniline.

2. A process as claimed in claim 1, characterized in that, before or after its separation into two partial streams, water is removed by distillation in a distillation stage (9) from the aqueous phase leaving the product extraction stage (8) in a quantity exceeding the quantity of water required to maintain a constant volume in the circuit and this quantity of water exceeding the quantity required to maintain a constant water volume is returned to the circuit at any point after the last reaction stage (6) and before the extraction stage (8).

3. A process as claimed in claim 2, characterized in that, before it is recycled, the water accumulating as distillate in the distillation stage (9) is at least partly used to wash the organic phase leaving the product extraction stage (8) and/or the organic phase leaving the post-extraction stage (10).

4. A process as claimed in claims 1 to 3, characterized in that the catalyst solution (C) to be returned to the beginning of the process is divided into two or more partial streams of which the first partial stream is returned to the first reaction stage (5) while the other partial streams are returned to the reaction mixture in the further course of the first reaction stage (5) and before the product extraction stage (8).

## Revendications

1. Procédé pour la préparation de polyamines aromatiques polynucléaires par la mise en réaction d'aniline avec du formaldéhyde en présence d'eau et de catalyseurs acides dans une réaction à une ou à deux étapes dans le domaine de température de 0 à 180°C, éventuellement en plaçant en amont une étape préliminaire de préparation d'aminal dans laquelle a lieu la formation de l'aminal N,N'-disubstitué que l'on transforme alors en une ou en plusieurs étapes en produit final désiré en présence d'un catalyseur acide dans le domaine de température de 0 à 180°C, par traitement du mélange réactionnel résultant en procédant à une extraction dans un solvant hydrophobe contenant de l'aniline dans une étape d'extraction de produit (8), par séparation par voie de distillation de la phase organique obtenue en l'occurrence en (i) un produit de distillation constitué par le solvant contenant de l'aniline, que l'on remet en oeuvre lors de l'extraction, éventuellement après addition d'aniline fraîche et en (ii) un résidu de distillation constitué essentiellement par le produit issu du procédé, et par recyclage de la phase aqueuse que l'on obtient lors de l'extraction et qui contient le catalyseur acide en réutilisant le catalyseur qui y est contenu, tout en éliminant l'eau de condensation que l'on obtient lors de la réaction de condensation et l'eau incorporée dans le système avec la solution aqueuse du formaldéhyde dans un séparateur d'eau monté à la suite de l'étape préliminaire de préparation d'aminal et monté en amont de la première étape réactionnelle et/ou dans un évaporateur monté à la suite de l'étape d'extraction, caractérisé en ce que
a) on amène à réagir par mélange, le formaldéhyde que l'on doit mettre en oeuvre sous la forme d'une solution aqueuse dans une étape préliminaire de préparation d'aminal (3), avec une phase organique constituée par de l'aniline, éventuellement par des produits de condensation d'aniline-formaldéhyde et par un solvant hydrophobe, et/ou dans la première étape réactionnelle (5) avec une phase organique constituée par de l'aniline, éventuellement par des produits de condensation d'aniline-formaldéhyde et par un solvant hydrophobe et avec une phase aqueuse recyclée contenant le catalyseur sous forme de sels d'amine,
b) on sépare le mélange réactionnel diphasique ainsi obtenu au terme de la réaction dans un séparateur de phases (7) monté en amont de l'étape d'extraction de produit (8), en une phase aqueuse et en une phase organique,
c) on extrait la phase organique que l'on obtient dans le séparateur de phases (7) dans une étape d'extraction ultérieure (10) montée à la suite de l'étape d'extraction de produit (8), dans une partie ou éventuellement dans la quantité totale de la phase aqueuse que l'on obtient dans la réaction d'extraction de produit (8) essentiellement libérée du produit réactionnel,
d) on renvoie la phase aqueuse enrichie avec le produit réactionnel de la phase organique que l'on obtient dans le séparateur de phases (7), que l'on obtient dans l'étape d'extraction ultérieure (10), complètement ou au moins partiellement dans le processus réactionnel au terme de la transformation (6) et avant l'étape d'extraction principale (8), et on recycle le résidu de la phase aqueuse subsistant éventuellement avant le terme de la transformation (6),
e) on renvoie au début du processus la phase organique que l'on obtient dans l'étape d'extraction ultérieure (10), constituée par de l'aniline éventuellement par des produits de condensation d'aniline-formaldéhyde et par le solvant hydrophobe, et on fait réagir conformément à a),
f) on extrait, dans l'étape d'extraction de produit (8) dans un solvant hydrophobe contenant de l'aniline, la phase aqueuse que l'on obtient dans le séparateur de phases (7) éventuellement après combinaison avec la phase aqueuse provenant de l'étape d'extraction ultérieure (10),
g) on sépare en deux courants partiels (C) et (H) la phase aqueuse que l'on obtient dans l'étape d'extraction de produit (8), dont une est renvoyée au début du processus et dont l'autre est acheminée à l'étape d'extraction ultérieure (10), ou bien on guide, dans l'étape d'extraction ultérieure (10), la phase aqueuse que l'on obtient dans l'étape d'extraction de produit (8) et ensuite, on la sépare en deux courants partiels (C) et (H) dont un est renvoyé au début du processus et dont l'autre est recyclé au terme de la transformation (6) et avant l'étape d'extraction principale (8),
h) on sépare, dans l'étape de distillation (11), la phase organique que l'on obtient dans l'étape d'extraction de produit (8), en un produit de distillation constitué par un solvant hydrophobe contenant de l'aniline et en un résidu de distillation constitué essentiellement par le produit issu du procédé, et
i) on utilise, dans l'étape d'extraction de produit (8), comme agent d'extraction, le produit de distillation que l'on obtient dans l'étape de distillation (11), après addition d'aniline fraîche.

2. Procédé selon la revendication 1, caractérisé en ce qu'on prélève par distillation, dans une étape de distillation (9), à la phase aqueuse quittant l'étape d'extraction de produit (8) avant ou après sa séparation en deux courants partiels, une quantité d'eau qui excède celle requise pour maintenir un volume d'eau constant dans le circuit, et on la renvoie dans le circuit à l'intervention de la quantité d'eau en excès pour le maintien constant du volume d'eau, à n'importe quel endroit après la dernière étape réactionnelle (6) et avant l'étape d'extraction (8).

3. Procédé selon la revendication (2), caractérisé en ce qu'on utilise l'eau que l'on obtient sous forme de produit de distillation dans l'étape de distillation (9), avant son recyclage, au moins partiellement pour laver la phase organique quittant l'étape d'extraction de produit (8) et/ou la phase organique quittant l'étape d'extraction ultérieure (10).

4. Procédé selon les revendications 1 à 3, caractérisé en ce qu'on répartit en deux ou plusieurs courants partiels la solution de catalyseur (C) à renvoyer au début du processus et on guide en retour le premier courant partiel dans la première étape réactionnelle (5) et les autres courants partiels dans le mélange réactionnel dans la poursuite ultérieure de la première étape réactionnelle (5) et avant l'étape d'extraction de produit (8).
